# EUROPEAN PATENT APPLICATION

(11) **EP 4 741 373 A1**
(43) Date of publication of application: **13.05.2026**
(21) Application number: 24835911.9
(22) Date of filing: 20.06.2024
(51) Int. Cl.: C07C 321/14, C10M 135/20, C10N 20/02, C10N 30/06, C10N 40/06, C10N 40/08, C10N 40/22, C10N 40/24, C10N 40/25

(54) **SULFIDE COMPOUND, EXTREME PRESSURE ADDITIVE, AND LUBRICANT OIL COMPOSITION CONTAINING SAID EXTREME PRESSURE ADDITIVE**

(30) Priority: 06.07.2023 JP 2023111322
(71) Applicant: DIC Corporation, Tokyo 174-8520 (JP)
(72) Inventor: MATSUEDA, Hironobu, Kamisu-shi, Ibaraki 314-0193 (JP)
(74) Representative: Kraus & Lederer PartGmbB
(86) International application number: PCT/JP2024/022333
(87) International publication number: WO 2025/009398

(57) **Abstract**

Provided is a sulfide compound capable of functioning as an extreme pressure agent excellent in oxidation stability and stability of a frictional wear reducing effect. Specifically, a sulfide compound containing at least a fatty acid triglyceride and sulfur as reaction components, wherein a content proportion of oleic acid in fatty acids constituting the fatty acid triglyceride is 80 mass% or more.

## Description

### TECHNICAL FIELD

The present invention relates to a sulfide compound, an extreme pressure agent, and a lubricant oil composition containing the extreme pressure agent.

### BACKGROUND ART

Lubricant oils such as gear oils, engine oils, hydraulic oils, cutting oils, plastic working oils, and casting oils are used by blending various additives such as extreme pressure agents, oiliness agents, antioxidants, rust inhibitors, corrosion inhibitors, antifoaming agents, and nonferrous metal corrosion inhibitors with base oils such as mineral oils, fats and oils, and synthetic oils.

Among the above additives, the extreme pressure agent is an additive for reducing frictional wear between metals, and various extreme pressure agents such as a chlorine-based extreme pressure agent, a sulfur-based extreme pressure agent, a phosphorus-based extreme pressure agent, and an organic metal compound-based extreme pressure agent have been proposed (for example, Patent Documents 1 to 3).

### CITATION LIST

### PATENT DOCUMENT

Patent Document 1: JP 2021-500244 A
Patent Document 2: WO2020/030189
Patent Document 3: WO2014/188948

### SUMMARY OF INVENTION

### TECHNICAL PROBLEM

Since the lubricant oil is generally exposed to high temperature and high pressure, there is a problem that the extreme pressure agent is oxidized and deteriorated, and thus that the intended effect cannot be obtained. Due to such a problem, oxidation stability is required also for the extreme pressure agent as an additive, but the extreme pressure agents of Patent Documents 1 to 3 cannot meet the required performance. In addition, the extreme pressure agent is required to stably exhibit a frictional wear reducing effect under various loads, but the stability of a frictional wear reducing effect of the extreme pressure agents of Patent Documents 1 to 3 cannot be said to be sufficient.

An object of the present invention is to provide a sulfide compound which has excellent oxidation stability and excellent stability of an effect of reducing frictional wear between metals, and can function as, for example, an extreme pressure agent.
Another object of the present invention is to provide an extreme pressure agent having excellent oxidation stability and excellent stability of the frictional wear reducing effect, and a lubricant oil composition containing the extreme pressure agent.

### SOLUTION TO PROBLEM

The present inventors have conducted intensive studies to solve the above problems, and, as a result, have found that a sulfide compound containing at least an oleic acid-rich unsaturated fatty acid and sulfur as reaction components is excellent in oxidation stability and frictional wear reducing effect stability, thereby completing the present invention.

That is, the present invention relates to the following sulfide compound and the like.
1. A sulfide compound containing at least a fatty acid triglyceride and sulfur as reaction components,
   wherein a content proportion of oleic acid in fatty acids constituting the fatty acid triglyceride is 80 mass% or more.
2. The sulfide compound according to 1, wherein a content proportion of linoleic acid in the fatty acids constituting the fatty acid triglyceride is 5 mass% or less.
3. The sulfide compound according to 1 or 2, wherein the fatty acid triglyceride is an algal oil.
4. The sulfide compound according to any one of 1 to 3, wherein a proportion of the sulfur in the reaction components is in a range of from 5 to 35 mass% relative to a total amount of the reaction components.
5. The sulfide compound according to any one of 1 to 4, further containing an unsaturated fatty acid alkyl ester as the reaction component.
6. The sulfide compound according to 5, wherein the unsaturated fatty acid alkyl ester is contained in an amount in a range of from 10 to 300 parts by mass relative to 100 parts by mass of the fatty acid triglyceride.
7. The sulfide compound according to any one of 1 to 6, wherein a kinematic viscosity at 40°C is in a range of from 100 to 1500 mm²/s.
8. The sulfide compound according to any one of 1 to 7, which is an extreme pressure agent.
9. A lubricant oil composition containing a base oil and the extreme pressure agent according to claim 8.

### ADVANTAGEOUS EFFECTS OF INVENTION

According to the present invention, it is possible to provide a sulfide compound which has excellent oxidation stability and excellent stability of an effect of reducing frictional wear between metals, and can function as, for example, an extreme pressure agent.
According to the present invention, it is possible to provide an extreme pressure agent having excellent oxidation stability and excellent stability of the frictional wear reducing effect, and a lubricant oil composition containing the extreme pressure agent.

### DESCRIPTION OF EMBODIMENTS

Hereinafter, an embodiment of the present invention will be described. The present invention is not limited by the following embodiments, and modification can be appropriately applied in a range that does not impair the effect of the present invention.
The compound in the present specification may be derived from fossil resources or may be derived from biological resources.

### Sulfide Compound

The sulfide compound of the present invention is a sulfide compound containing at least a fatty acid triglyceride and sulfur as reaction components, wherein a content proportion of oleic acid in fatty acids constituting the fatty acid triglyceride is 80 mass% or more.

The sulfide compound of the present invention includes a compound containing fatty acid triglycerides crosslinked with each other by sulfur, in which sulfur forms a crosslinked structure at a carbon-carbon double bond portion derived from an unsaturated fatty acids constituting the fatty acid triglycerides. In the present invention, oleic acid, which is a monovalent unsaturated fatty acid, constitutes 80 mass% or more of the fatty acids constituting the fatty acid triglyceride, whereby sulfur is likely to undergo intermolecular crosslinking, and a sulfide compound having a long aliphatic chain and a small number of carbon-carbon unsaturated bonds can be obtained, and the oxidation stability is excellent. In addition, when oleic acid, which is an unsaturated fatty acid, constitutes 80 mass% or more of the fatty acids, a proportion of the saturated fatty acid is relatively reduced, and the sulfide compound of the present invention has good fluidity and can exhibit a stable frictional wear reducing effect.
Hereinafter, the reaction components of the sulfide compound and the like will be described.

The fatty acid triglyceride is a triester obtained by esterifying each of three hydroxy groups of glycerin with a fatty acid, and is preferably an ester of a fatty acid having from 4 to 30 carbon atoms and glycerin. The fatty acid is preferably a fatty acid having from 8 to 30 carbon atoms, and more preferably a fatty acid having from 8 to 22 carbon atoms.
The fatty acid may be either a saturated fatty acid or an unsaturated fatty acid, but oleic acid constitutes 80 mass% or more of the fatty acid.

In the present invention, a content proportion of the oleic acid in the fatty acids constituting the fatty acid triglyceride is 80 mass% or more, preferably 85 mass% or more, more preferably 87 mass% or more, and further preferably 90 mass% or more.
An upper limit of the content proportion of the oleic acid is not particularly limited, and is, for example, 95 mass% or less, 99 mass% or less, or 100 mass% or less.

A content proportion of linoleic acid in the fatty acids constituting the fatty acid triglyceride is preferably 8 mass% or less, more preferably 5 mass% or less, and further preferably 3 mass% or less.
By setting a content of the linoleic acid, which is a divalent unsaturated fatty acid, within the above range, an amount of carbon-carbon unsaturated bonds in the sulfide compound can be suppressed, and the oxidation stability can be enhanced.
A lower limit of the content of the linoleic acid in the unsaturated fatty acid is not particularly limited, and is, for example, 0 mass% or more, 0.1 mass% or more, or 1 mass% or more.

Contents of various fatty acids such as oleic acid, linoleic acid, palitimic acid, stearic acid, linolenic acid, lauric acid, behenic acid, montanic acid, butanoic acid, pentanoic acid, hexanoic acid, decanoic acid, myristic acid, eicosenoic acid, erucic acid, stearidonic acid, punicic acid, palmitoleic acid, ricinoleic acid, eicosadienoic acid, docosadienoic acid, eleostearic acid, pinolenic acid, 9,11-octadecadienoic acid, and 9,12-octadecadienoic acid constituting the fatty acid triglyceride are confirmed by the method described in the Examples.

The fatty acid triglyceride is preferably an algal oil.
The fatty acid triglyceride derived from algal oil is generally a fatty acid triglyceride rich in oleic acid, and the fatty acid triglyceride derived from algal oil can be easily adjusted to have an oleic acid content of 80 mass% or more and a linoleic acid content of 8 mass% or less.

Algae (organisms that perform oxygen-generating photosynthesis) store lipids in their cells, and the term "algal oil" as used herein means an oil extracted from algae. As used herein, the term "algae" includes eubacteria, eukaryotic unicellular organisms and eukaryotic multicellular organisms.

Examples of the algae which are eubacteria include species of the genus *Spirulina,* and species of the genus *Arthrospira.*

Examples of the algae which are eukaryotic unicellular organisms and eukaryotic multicellular organisms include species of the genus *Chlorella,* species of the genus *Pseudochlorella,* species of the genus *Heterochlorella,* species of the genus *Prototheca,* species of the genus *Arthrospira,* species of the genus *Euglena,* species of the genus *Nannochloropsis,* species of the genus *Phaeodactylum,* species of the genus *Chlamydomonas,* species of the genus *Scenedesmus,* species of the genus *Ostreococcus,* species of the genus *Selenastrum,* species of the genus *Haematococcus,* species of the genus *Nitzschia,* species of the genus *Dunaliella,* species of the genus *Navicula,* species of the genus *Trebouxia,* species of the genus *Pseudotrebouxia,* species of the genus *Vavicula,* species of the genus *Bracteococcus,* species of the genus *Gomphonema,* species of the genus *Watanabea,* species of the genus *Botryococcus,* species of the genus *Tetraselmis,* and species of the genus *Isochrysis.*

The algae can produce fatty acid triglycerides having a desired composition by, for example, recombination of fatty acid synthesis genes, and the algae in the present specification include also genetically modified products of the above-described algae.

As the fatty acid triglyceride, not only the fatty acid triglyceride derived from algal oil but also a fatty acid triglyceride derived from animal oil and a fatty acid triglyceride derived from vegetable oil are known. Specifically, fatty acid triglycerides derived from lard, beef tallow, fish oil, rapeseed oil, coconut oil, canola oil, coconut oil, corn oil, cottonseed oil, olive oil, palm oil, peanut oil, sunflower oil, soybean oil, safflower oil, rice oil, palm oil, sesame oil, linseed oil, grape seed oil, and the like are known.

However, it is difficult to make the content of the oleic acid constituting the fatty acid triglyceride 80 mass% or more in both the fatty acid triglyceride derived from animal oil and the fatty acid triglyceride derived from vegetable oil. As the fatty acid triglyceride derived from animal oil, for example, a fatty acid triglyceride derived from lard has an oleic acid content of less than 50 mass% and is a fatty acid triglyceride having a relatively large amount of saturated fatty acid components. In addition, as the fatty acid triglyceride derived from vegetable oil, a fatty acid triglyceride derived from palm oil has an oleic acid content of less than 50 mass%, and a fatty acid triglyceride derived from canola oil also has an oleic acid content of less than 65 mass%.

A proportion of sulfur in the reaction components is, for example, in a range of from 5 to 35 mass%, preferably in a range of from 5 to 25 mass%, more preferably in a range of from 5 to 20 mass%, and further preferably in a range of from 10 to 17 mass% or in a range of from 8 to 15 mass%, relative to a total amount of the reaction components.

The reaction components of the sulfide compound of the present invention may contain sulfur and a fatty acid triglyceride, and may contain a component other than the sulfur and the fatty acid triglyceride.

The reaction components other than the sulfur and the fatty acid triglyceride include an alkyl ester of an unsaturated fatty acid. By using an unsaturated fatty acid alkyl ester as the reaction component, the sulfide compound of the present invention includes a compound in which unsaturated fatty acid alkyl esters are crosslinked with each other by sulfur, and the kinematic viscosity of the resulting sulfide compound can be adjusted.

The compound in which unsaturated fatty acid alkyl esters are crosslinked with each other by sulfur is, for example, a compound represented by General Formula (1) below. 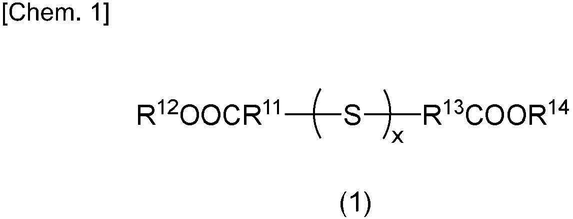 In General Formula (1),
R¹¹ and R¹³ are each independently an alkylene group having from 3 to 29 carbon atoms or a divalent hydrocarbon group having from 3 to 29 carbon atoms and having one or more carbon-carbon unsaturated bonds, and
R¹² and R¹⁴ are each independently an alkyl group having from 1 to 12 carbon atoms.

The compound represented by General Formula (1) has a structure in which unsaturated fatty acid alkyl esters corresponding to R¹¹COOR¹² and R¹³COOR¹⁴ are linked by a (poly) sulfide bond (x is, for example, an integer of from 1 to 8) at a carbon-carbon unsaturated bond portion.

In General Formula (1), the alkylene group having from 3 to 29 carbon atoms of R¹¹ and R¹³ may be linear or branched.
The alkylene group having from 3 to 29 carbon atoms of R¹¹ and R¹³ is preferably an alkylene group having from 7 to 21 carbon atoms.

In General Formula (1), the "divalent hydrocarbon group having from 3 to 29 carbon atoms and one or more carbon-carbon unsaturated bonds" of R¹¹ and R¹³ is an alkylene group having one or more carbon-carbon unsaturated bonds, such as an alkenylene group, and may be linear or branched.
The divalent hydrocarbon group having from 3 to 29 carbon atoms and one or more carbon-carbon unsaturated bonds of R¹¹ and R¹³ is preferably a divalent hydrocarbon group having from 7 to 21 carbon atoms and one or more carbon-carbon unsaturated bonds.

In General Formula (1), the alkyl having from 1 to 12 carbon atoms of R¹² and R¹⁴ may be linear or branched.
The alkyl group having from 1 to 12 carbon atoms of R¹² and R¹⁴ is preferably an alkyl group having from 1 to 6 carbon atoms.

As the unsaturated fatty acid alkyl ester, for example, an ester of an unsaturated fatty acid having from 4 to 30 carbon atoms and an alcohol having from 1 to 12 carbon atoms can be used. The unsaturated fatty acid having from 4 to 30 carbon atoms is preferably an unsaturated fatty acid having from 8 to 22 carbon atoms, and the alcohol having from 1 to 12 carbon atoms is preferably an alcohol having from 1 to 6 carbon atoms.

Specific examples of the unsaturated fatty acid having from 4 to 30 carbon atoms include oleic acid, linoleic acid, linolenic acid, eicosenoic acid, erucic acid, stearidonic acid, punicic acid, palmitoleic acid, ricinoleic acid, eicosadienoic acid, docosadienoic acid, eleostearic acid, pinolenic acid, 9,11-octadecadienoic acid, and 9,12-octadecadienoic acid.

The alkyl ester of an unsaturated fatty acid can be prepared by decomposing a fatty acid triglyceride into glycerin and a fatty acid with, for example, sodium hydroxide, and esterifying the obtained fatty acid with an alcohol. The unsaturated fatty acid alkyl esters obtained by esterifying fatty acids obtained by decomposing fatty acid triglycerides with alcohols are preferred because common raw materials can be used.
The fatty acid preferably has an oleic acid content of 80 mass% or more. The fatty acid preferably has a linoleic acid content of 8 mass% or less.

An amount of the alkyl ester of an unsaturated fatty acid to be used is, for example, from 10 to 300 parts by mass, preferably from 50 to 200 parts by mass, and more preferably from 70 to 150 parts by mass, relative to 100 parts by mass of the fatty acid triglyceride.

As the reaction component other than the sulfur and the fatty acid triglyceride, an unsaturated hydrocarbon can also be used. By using an unsaturated hydrocarbon as the reaction component, the sulfide compound of the present invention includes a compound in which unsaturated hydrocarbons are crosslinked with each other by sulfur, and the kinematic viscosity of the resulting sulfide compound can be adjusted.

The compound in which unsaturated hydrocarbons are crosslinked with each other by sulfur is, for example, a compound represented by General Formula (2) below. 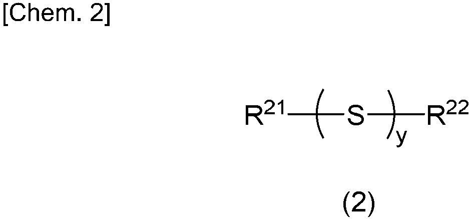 In General Formula (2),
R²¹ and R²² are each independently an alkyl group having from 4 to 30 carbon atoms or a hydrocarbon group having from 4 to 30 carbon atoms and having one or more carbon-carbon unsaturated bonds.

The compound represented by General Formula (2) has a structure in which unsaturated hydrocarbons corresponding to R²¹ and R²² are linked by a (poly) sulfide bond (y is, for example, an integer of from 1 to 8) at a carbon-carbon unsaturated bond portion.

In General Formula (2), the alkyl group having from 4 to 30 carbon atoms of R²¹ and R²² may be linear or branched.
The alkyl group having from 4 to 30 carbon atoms of R²¹ and R²² is preferably an alkyl group having from 4 to 22 carbon atoms.

In General Formula (1), the "hydrocarbon group having from 4 to 30 carbon atoms and one or more carbon-carbon unsaturated bonds" of R²¹ and R²² is a hydrocarbon group having one or more carbon-carbon unsaturated bonds, such as an alkenyl group, and may be linear or branched.
The hydrocarbon group having from 4 to 30 carbon atoms and one or more carbon-carbon unsaturated bonds of R²¹ and R²² is preferably a hydrocarbon group having from 4 to 22 carbon atoms and one or more carbon-carbon unsaturated bonds.

The unsaturated hydrocarbon may be an unsaturated hydrocarbon having at least one carbon-carbon unsaturated bond, and is preferably an unsaturated hydrocarbon having from 4 to 30 carbon atoms, and more preferably an unsaturated hydrocarbon having from 4 to 22 carbon atoms. Specific examples of the unsaturated hydrocarbon include 1-butene, 1-pentene, 1-hexene, 1-octene, 1-decene, 1-dodecene, 1-tetradecene, 1-hexadecene, 1-octadecene, 1-eicosene, 3-methylpentene, 4-methylheptene, 5-methylundecene, 3,6-dimethylhexanedecene, and diisobutylene.

The unsaturated hydrocarbon may be derived from, for example, mineral oil.

An amount of the unsaturated hydrocarbon to be used is, for example, from 10 to 300 parts by mass, preferably from 50 to 200 parts by mass, and more preferably from 70 to 150 parts by mass, relative to 100 parts by mass of the fatty acid triglyceride.

Fatty acid glycerides from animal oil, vegetable oil, algal oil and the like are usually present as triglycerides in which three fatty acids are bonded to glycerin, but may also include fatty acid monoglycerides and fatty acid diglycerides as the reaction components within a range that does not impair the effects of the present invention.
When the fatty acid monoglyceride and/or the fatty acid diglyceride are contained as the reaction component(s), a total of the fatty acid monoglyceride and the fatty acid diglyceride may be, for example, 10 mass% or less, 5 mass% or less, or 3 mass% or less of the total amount of the reaction components.

The reaction components of the sulfide compounds of the present invention may consist substantially of, for example, sulfur, a fatty acid triglyceride, any unsaturated fatty acid alkyl ester, and any unsaturated hydrocarbon. The term "consists substantially of" means that a total content of sulfur, a fatty acid triglyceride, any unsaturated fatty acid alkyl ester, and any unsaturated hydrocarbon is 90 mass% or more, 95 mass% or more, 98 mass% or more, or 100 mass% of the total amount of the reaction components of the sulfide compound of the present invention.

A kinematic viscosity at 40°C of the sulfide compound of the present invention is, for example, in a range of from 100 to 1500 mm²s, preferably in a range of from 100 to 500 mm²s. The kinematic viscosity at 40°C of the sulfide compound is confirmed by the method described in the Examples.

### Method for Producing Sulfide Compound

The sulfide compound of the present invention can be produced by a known method, and can be prepared, for example, by reacting sulfur, a fatty acid triglyceride, any unsaturated fatty acid alkyl ester, and any unsaturated hydrocarbon in the presence of hydrogen sulfide and a basic catalyst.

The nature of sulfur at the time of producing the sulfide compound of the present invention is not particularly limited, and may be a solid state such as a small lump shape, a flake shape, or a powder shape, or may be a molten state (liquid). In view of ease of increasing a production scale, sulfur is preferably in a molten state.

Examples of the basic catalyst include alkali metal hydroxides (for example, sodium hydroxide and potassium hydroxide), an aliphatic amine compound, and an aromatic amine compound.
An amount of the basic catalyst to be used is, for example, from 0.05 to 1.0 parts by mass relative to 100 parts by mass of the reaction components.

A reaction temperature may be set in a range of, for example, from 50 to 200°C, and a reaction time may be set in a range of, for example, from 1 to 72 hours.

### Lubricant Oil Composition

The sulfide compound of the present invention can be suitably used as an extreme pressure agent. Here, the "extreme pressure agent" is an additive that suppresses wear due to friction between metals, and it is considered that the sulfide compound is adsorbed on a metal surface to form a lubricating film (tribofilm), thereby suppressing wear.
The extreme pressure agent which is the sulfide compound of the present invention (hereinafter sometimes referred to as "extreme pressure agent of the present invention") has excellent oxidation stability, and therefore, deterioration due to heat generated by friction between metals is reduced, and generation of sludge can be suppressed.

The lubricant oil composition of the present invention contains a lubricating base oil and the extreme pressure agent of the present invention. As the lubricating base oil, known ones can be used, and examples thereof include mineral oils such as distillate oils or refined oils of paraffin group-based crude oils, intermediate group-based crude oils, and naphthene group-based crude oils; and synthetic oils such as low molecular weight polybutene, low molecular weight polypropylene, α-olefin oligomers having from 8 to 14 carbon atoms, hydrides of α-olefin oligomers having from 8 to 14 carbon atoms, polyol esters (fatty acid esters of trimethylolpropane, fatty acid esters of pentaerythritol, and the like), ester compounds (dibasic acid esters, aromatic polycarboxylic acid esters, phosphoric acid esters, and the like), alkyl aroma compounds (alkylbenzenes, alkylnaphthalenes, and the like), polyglycol oils (polyalkylene glycols, and the like), and silicone oils.

The lubricating base oil may be appropriately selected depending on the purpose of use, the conditions of use, and the like, and one type of the lubricating base oil may be used alone or two or more types thereof may be used in combination.

A content of the extreme pressure agent of the present invention in the lubricant oil composition of the present invention is, for example, from 0.01 to 50 parts by mass, preferably from 1 to 50 parts by mass, and more preferably from 1 to 30 parts by mass, relative to 100 parts by mass of the lubricating base oil.

One type of the extreme pressure agent of the present invention may be used alone, or two or more types thereof may be used in combination.

The lubricant oil composition of the present invention may contain a lubricating base oil and the extreme pressure agent of the present invention, and may further contain an additive. Examples of the additive include oiliness agents, antiwear agents, extreme pressure agents other than the extreme pressure agent of the present invention, rust inhibitors, corrosion inhibitors, antifoaming agents, detergent-dispersants, pour point depressants, viscosity index improvers, antioxidants, emulsifiers, demulsifiers, mold inhibitors, friction modifiers, and surfactants.
Any of these additives may be a known additive.

The lubricant oil composition of the present invention may be used as a grease by further incorporating a thickener such as a metal soap.

The use of the lubricant oil composition of the present invention is not particularly limited, and examples thereof include automotive lubricant oils used for internal combustion engines, automatic transmissions, shock absorbers, drive system devices such as power steering, gears, and the like; metal working oils used for metal working such as cutting, grinding, and plastic working; and hydraulic oils which are power transmission fluids used for operations such as power transmission, force control, and shock absorption in hydraulic systems such as hydraulic devices and apparatuses.

### EXAMPLES

The present invention will be specifically described with reference to Examples and Comparative Examples below.
Note that the present invention is not limited to the following examples.

### Example 1: Preparation of Sulfide Compound (1)

In a 1-L autoclave equipped with a warming apparatus, a hydrogen sulfide blowing tube, and a hydrogen sulfide absorber, 300 g of a fatty acid triglyceride (fatty acids constituting the fatty acid triglyceride: 94 mass% of oleic acid and less than 2 mass% of linoleic acid), 300 g of methyl ester of the fatty acid triglyceride (obtained by decomposing the fatty acid triglyceride into glycerin and a fatty acid with sodium hydroxide, and esterifying the obtained fatty acid with methanol), 52 g of sulfur, and 3.8 g of a mixture of alkylamines having from 16 to 22 carbon atoms as a catalyst were charged.
The autoclave was sealed, and the pressure in the reactor was reduced to - 0.1 MPa or less by using a vacuum pump, followed by vacuum degassing. Thereafter, the mixture was heated until the internal temperature reached 100°C. Here, 12 g of hydrogen sulfide gas (purity: 99.9 mol%) was blown at a pressure of 6 kg/cm² for 1 hour, and the temperature was raised to 120°C. Thereafter, 18 g of hydrogen sulfide gas was blown over 3.5 hours and retained at 120°C for 5 hours, and then the temperature was raised to 150°C and retained for 4 hours. Thereafter, the reaction mixture was cooled to 80°C, the pressure was returned to atmospheric pressure by opening a valve connected to the hydrogen sulfide absorber, air was blown through the blowing tube to distill off the remaining hydrogen sulfide, and 3.5 g of a thiadiazole and 0.5 g of a benzotriazole were added to obtain 669 g of a sulfide compound (1) (yield: 97%, kinematic viscosity at 40°C: 490 mm²/s, sulfur content: 11.0 mass%).

The fatty acid triglyceride used in the preparation of the sulfide compound (1) was an algal oil derived from chlorella (available from Checkerspot Inc.), and the proportions of the components of the fatty acids constituting the fatty acid triglyceride were identified using gas chromatography. Specifically, the algal oil was decomposed into glycerin and fatty acids with sodium hydroxide to extract the fatty acids, the extracted fatty acids were methyl-esterified with methanol, and the obtained fatty acid methyl esters were subjected to gas chromatography to identify various fatty acids.

The algal oil is merely a "fatty acid triglyceride extracted from algae" and does not substantially contain components other than the fatty acid triglyceride. Therefore, the fatty acid triglyceride can be artificially reproduced by reacting glycerin with the fatty acid in the above blending ratio.

### Comparative Example 1: Preparation of Sulfide Compound (1')

A sulfide compound (1') (669 g) (yield: 97%, kinematic viscosity at 40°C: 330 mm²/s, sulfur content: 11.5 mass%) was obtained in the same manner as in Example 1 except that canola oil (triglyceride of 63 mass% of oleic acid and 19 mass% of linoleic acid) was used instead of the algal oil, and that methyl esters of unsaturated fatty acids (82 mass% of oleic acid and 9 mass% of linoleic acid) were used instead of the methyl esters of the algal oil.

### Comparative Example 2: Preparation of Sulfide Compound (2')

In a 1-L autoclave equipped with a warming apparatus, a hydrogen sulfide blowing tube, and a hydrogen sulfide absorber, 300 g of lard (triglyceride of 49 mass% of oleic acid and 10 mass% of linoleic acid), 184 g of unsaturated fatty acid methyl esters (oleic acid: from 40 to 50%, linoleic acid: from 25 to 35%) of a commercially available recovered oil, 48 g of sulfur, and 1 g of a mixture of alkylamines having from 16 to 22 carbon atoms as a catalyst were charged. The autoclave was sealed, and the pressure in the reactor was reduced to - 0.1 MPa or less by using a vacuum pump, followed by vacuum degassing. Thereafter, the mixture was heated until the internal temperature reached 100°C. Here, 7.5 g of hydrogen sulfide gas (purity: 99.9 mol%) was blown at a pressure of 6 kg/cm² over 1 hour, and the temperature was raised to 120°C. Thereafter, 11.2 g of hydrogen sulfide gas was blown over 3.5 hours and retained at 120°C for 5 hours, and then the temperature was raised to 165°C and retained for 6 hours. Thereafter, the reaction mixture was cooled to 80°C, the pressure was returned to atmospheric pressure by opening a valve connected to the hydrogen sulfide absorber, air was blown through the blowing tube to distill off the remaining hydrogen sulfide, and 3.5 g of a thiadiazole and 0.4 g of a benzotriazole were added to obtain 539 g of a sulfide compound (2') (yield: 97%, kinematic viscosity at 40°C: 320 mm²/s, sulfur content: 11.5 mass%).

The sulfide compounds produced in the Examples and the Comparative Examples were evaluated as follows. The results are presented in Table 1.

### Sulfur Content

The sulfur content of the sulfide compound was measured based on JIS K2541-7:2023.

### Kinematic Viscosity

The kinematic viscosity of the sulfide compound at 40°C was measured based on JIS K2283:2000. A higher kinematic viscosity means more excellent handleability of the sulfide compound.

### Pour Point

The pour point of the sulfide compound was measured based on JIS K2269:1987. A lower pour point means that the fluidity is retained even at a low temperature, and means that the handleability of the sulfide compound is more excellent.

### Oxidation Stability

A lubricant oil composition was prepared by adding each of the sulfide compounds produced in the Examples and Comparative Examples to a Group II oil (kinematic viscosity at 40°C: 90 mm²/s) so that the content of the sulfide compound was 5 mass%. The results of the following evaluation of acid value stability using the prepared lubricant oil composition are shown in Table 1.

The oxidation stability of the sulfide compound was evaluated based on JIS K 2514:2013. Specifically, the lubricant oil composition was deteriorated under conditions of 135°C and 96 hours using an ISOT tester equipped with a glass varnish rod. After the completion of the test, the presence or absence of sludge adhesion to the varnish rod was visually evaluated according to the following criteria.
○: Sludge adhering to the varnish rod was hardly observed
×: Sludge adhering to the varnish rod can be clearly confirmed

### Extreme Pressure Performance

A lubricant oil composition was prepared by adding each of the sulfide compounds produced in the Examples and Comparative Examples to paraffin oil (kinematic viscosity at 40°C: 9 mm²/s) so that the content of the sulfide compound was 5 mass%. The results of the following evaluation of extreme pressure performance using the prepared lubricant oil composition are shown in Table 1.

The extreme pressure performance of the sulfide compound was evaluated in terms of fusion load and maximum non-seizure load according to ASTM D-2783. Specifically, the extreme pressure performance of the lubricant oil composition was evaluated under the following conditions.
Vertical shaft rotation speed: 1770 rpm
Test steel ball: 1/2 inch (SUJ2) for ball bearing
Measurement time of fusion load and maximum non-seizure load: 10 seconds

### Friction Characteristics

The changes in coefficient of friction of the sulfide compounds produced in the Examples and Comparative Examples were evaluated according to ASTM D2596. In particular, a four ball tester (TE92, available from Plint) was used as a tester, and an SUJ2 carbon ball having a diameter of 1/2 inch was used as a test ball, and a load in a range of from 500 to 3000 N was applied stepwise every 120 seconds while rotating the test ball at 200 rpm, and the maximum torque value during rotation was observed.
The coefficient of friction was calculated from the obtained torque values according to the calculation formula: coefficient of friction = maximum torque value (N·cm)/(1.65 × vertical load per ball) (N), and the change in coefficient of friction was evaluated according to the following criteria.
As the change in coefficient of friction occurs on a higher load side, the corrosive wear due to frictional heat can be reduced.
×: A µ change of 0.07 or more occurs at a load of less than 1000 N.
△: A µ change of 0.07 or more occurs at a load of 1000 N or more and less than 2000 N
○: A µ change of 0.07 or more occurs at a load of 2000 N or more and 3000 N or less

**[Table 1]**

| | Example 1 | Comparative Example 1 | Comparative Example 2 |
|---|---|---|---|
| Sulfur content [mass%] | 11.0 | 11.5 | 11.5 |
| Kinematic viscosity at 40°C [mm2/s] | 490 | 330 | 320 |
| Pour point [°C] | -20 | -10 | 17.5 |
| Oxidation stability | ○ | × | ○ |
| Fusion load [kgf] | 250 | 240 | 250 |
| Maximum non-seizure load [kgf] | 63 | 63 | 63 |
| Friction characteristics | ○ | × | △ |

It is found that the sulfide compound of Example 1 using a fatty acid triglyceride having an oleic acid content proportion of 80 mass% or more is excellent in both oxidation stability and friction characteristics. On the other hand, the sulfide compound of Comparative Example 1 using a fatty acid triglyceride having an oleic acid content proportion of less than 80 mass% is significantly inferior in oxidation stability. In addition, the sulfide compound of Comparative Example 2 has poor friction characteristics because the amount of saturated fatty acid in the fatty acid triglyceride is relatively large.

## Claims

1. A sulfide compound comprising at least a fatty acid triglyceride and sulfur as reaction components,
wherein a content proportion of oleic acid in fatty acids constituting the fatty acid triglyceride is 80 mass% or more.

2. The sulfide compound according to claim 1, wherein a content proportion of linoleic acid in the fatty acids constituting the fatty acid triglyceride is 5 mass% or less.

3. The sulfide compound according to claim 1 or 2, wherein the fatty acid triglyceride is an algal oil.

4. The sulfide compound according to claim 1 or 2, wherein a proportion of the sulfur in the reaction components is in a range of from 5 to 35 mass% relative to a total amount of the reaction components.

5. The sulfide compound according to claim 1 or 2, further comprising an unsaturated fatty acid alkyl ester as the reaction component.

6. The sulfide compound according to claim 5, wherein the unsaturated fatty acid alkyl ester is contained in an amount in a range of from 10 to 300 parts by mass relative to 100 parts by mass of the fatty acid triglyceride.

7. The sulfide compound according to claim 1 or 2, wherein a kinematic viscosity at 40°C is in a range of from 100 to 1500 mm²/s.

8. The sulfide compound according to claim 1 or 2, wherein the sulfide compound is an extreme pressure agent.

9. A lubricant oil composition comprising a base oil and the extreme pressure agent described in claim 8.
